# EUROPEAN PATENT APPLICATION

(11) **EP 1 790 734 A1**
(43) Date of publication of application: **30.05.2007**
(21) Application number: 05025721.1
(22) Date of filing: 25.11.2005
(51) Int. Cl.: C12Q 1/42, C12Q 1/10

(54) **Microbial assay**

(71) Applicant: Biosynth AG, 9422 Staad (CH)
(72) Inventor: Spitz, Urs, 8704 Herrliberg ZH (CH)
(74) Representative: Felder, Peter

(57) **Abstract**

A method of detecting Shigella apyrase enzyme comprises the steps of: a) contacting a sample suspected of containing said Shigella apyrase enzyme with an endo-pyrophosphatase substrate comprising an endo pyrophosphate group that is susceptible to cleavage by said enzyme, said cleavage yielding a signalogen species comprising a monophosphate group, said signalogen species being susceptible to phosphatase-induced formation of a signalophore species; b) providing a phosphatase activity so as to convert said signalogen species into a signalophore species; c) monitoring for a signal from said signalophore species as an indication of the presence of said Shigella apyrase enzyme.
The assay can be used for the detection of Shigella spp. on enteroinvasive E. coli.

## Description

### FIELD OF THE INVENTION

The present invention relates to a method and a kit for detecting Shigella apyrase enzyme, to an assay method for detection of the presence of *Shigella spp.* or enteroinvasive *E. coli* (henceforth abbreviated as "EIEC") and to a use of an endo-pyrophosphatase enzyme substrate.

### BACKGROUND OF THE INVENTION

Detection of enzyme activity is important in clinical diagnostics and testing applications as well as in biochemistry, molecular biology, enzymology and histology research. The presence of certain enzymes in a sample is indicative for the presence of certain microbes. Hence, the detection of enzyme activity is helpful in microbial taxonomy.

Detection of enzyme activity can be used for isolation of bacteria on chromogenic or fluorogenic plating media. Colonies growing on such media become distinguishable if growth is associated with production of a detectable enzyme. Based on differentiating features such as color or fluorescence, individual colonies can be selected for further culturing or investigation.

Detection of enzyme activity can also help to detect and identify and to isolate microbial pathogens. Detection, identification and isolation of microbial pathogens may be based on taxonomy or detection of enzyme activity related to microbial virulence.

Enzymes are often involved in microbial pathogenesis. Some enzymes act as virulence factors while others are located on genetic virulence factors and are expressed by pathogenic microbes. Therefore, enzyme substrates can be used to detect the expression of virulence factors and serve to detect, identify and isolate virulent microbes.

Microbes that are similar or even belonging to the same species may occur in varieties that express virulence factors while others do not. Depending on whether they express a virulence factor or not, closely related microbes may be pathogenic or harmless. Detection of enzyme activity can be used for differentiating between them.

*Shigella spp.* and enteroinvasive *E. coli* are very dangerous microbial pathogens that cause Shigellosis in humans.

*Shigella spp.* and EIEC are known to produce Shigella apyrase enzymes. Shigella apyrase is associated with Shigella and EIEC virulence.

Enzyme substrate molecules useful for the detection of enzyme activity often consist of a combination of certain chemical substructures having specific functionalities.

One type of substructure serves to bind to certain enzymes, which will also be called "detectable enzymes". This type of substructure will be called "binding motif" (denoted as BM in Scheme 1).

A second type of substructure serves to produce detectable signal. This type of substructure will be called "signalogenic substructure" (denoted as SG in Scheme 1).

A third type of substructure is labile to the action of a detectable enzyme. This type of substructure will be called "enzyme labile group" (denoted as ELG in Scheme 1).

The above mentioned substructures may overlap and occasionally are combined into one and the same substructure having multiple functions.

Upon enzymatic transformation of the enzyme labile group by a detectable enzyme, the signalogen is activated, thus forming what will be called an "active signalogen" (denoted as aSG in Scheme 1).

Active signalogens generate so-called "signalophores" (denoted as SP in Scheme 1) either spontaneously or by the effect of chemical agents, reagent enzymes, irradiation or electric fields.

Generation of signalophores from active signalogens often includes deprotonation, oxidation, hydrolysis, metal complexation or other chemical reactions. These reactions may involve chemical reagents as well as reagent enzymes.

The active signalogen and the signalophore may be of different origin, in which case the active siganolgen acts as an agent needed to generate the signalophore from a reagent precursor.

Typically, detectable signal (denoted as DS in Scheme 1) associated with the signalophore is released during this process; it may be of transient or persisting nature and it may occur spontaneously or it may require appropriate interrogation means such as optical excitation.

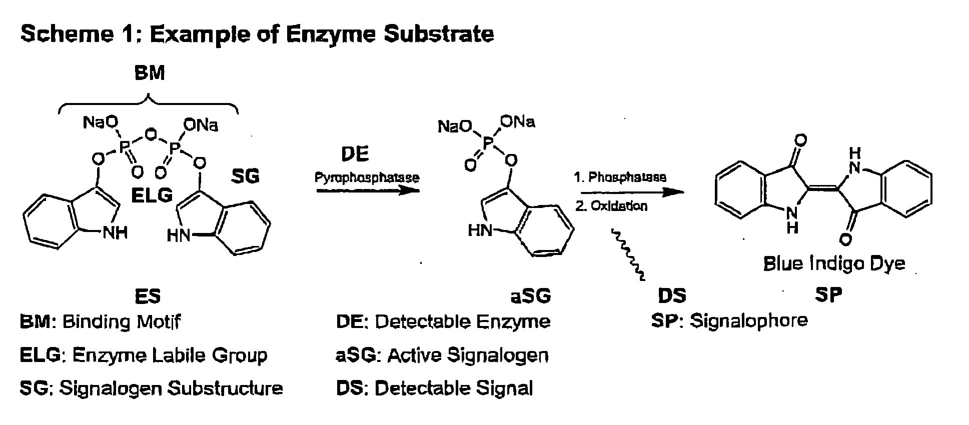

Detectable signals include changes in optical transmission (chromogenic substrates), changes in fluorescence (fluorogenic substrates, substrates for fluorescence resonant energy transfer FRET) emission of light (luminogenic substrates), changes in electric current or electric potential (electrogenic substrates) or any combination thereof.

Based on the nature of the signalogen, substrates are divided into different types as shown in Table1:

**Table 1**

| Detectable signal (DS) | Signalogen (SG) | Signalophore (SP) | Action of detected enzyme | Reference |
|---|---|---|---|---|
| Color | Chromogen | Chromophore | Active chromogen Is released | [1-3] |
| Fluorescence | Fluorogen | Fluoraphore | Active fluorogen is released | [1-3] |
| Fluorescence | Fluorophore and fluorescence quencher | Fluorophore | Quencher and fluorophore separate | [1-3] |
| Luminescence | Luminogen | Bio-, chemiluminescent transient molecule | Active luminogen is released | [4] |
| Electric current / potential | Electrogen | Oxidant, reductant | Active electrogen is released | [5] |

### References:

1. Goddard JP and Reymond JL: Recent advances in enzyme assays; Trends in Biotechnology, 2004 22, 363-370.
2. Manafi M: New developments in chromogenic and fluorogenic culture media; International Journal of Food Microbiology, 2000, 60, 205-218.
3. Restaino L, Frampton EW, Irbe RM, Schabert G and Spitz H: Isolation and Detection of Listeria monocytogenes using fluorogenic and chromogenic Substrates for phosphatidylinositol-specific phospholipase C; Journal of Food Prot. 1999, 62, 244-251.
4. Giri BP: Novel stabilized formulations for chemiluminescent assays; PCT/US01/02779.
5. Yeo WS and Mrksich M: Self-Assembled Monolayers That Transduce Enzymatic Activities to Electrical Signals; Angew. Chem. Int. Ed., 2003, 43, 3121-3104: Drummond TG, Hill MG and Barton JK: Electrochemical DNA sensors, Nature Biotechnology, 2003, 21, 1192-1199.

Shigella apyrase was first reported in 1995, when previously unknown enzyme activity that hydrolyzes nucleoside triphosphates was found in the periplasm of *Shigella spp.* (Bhargava T, Datta S, Ramachandran V, Ramakrishnan R, Roy RK, Sankaran K and Subrahmanyam YVBK: Virulent Shigella codes for a soluble apyrase: identification, characterization and cloning of the gene; Current Science, 1995, 68, 293-300; Babu MM. Kamalakkannana S, Subrahmanyam YVBK, Sankaran K.: Shigella apyrase - a novel variant of bacterial acid phosphatases? FEBS Letters, 2002, 512, 8-12; Santapaola D, Casalino M, Petrucca A, Presutti C, Zagaglia C, Berlutti F, Colonna B and Nicoletti M: Enteroinvasive Escherichia coli virulence-plasmid-carried apyrase (apy) and ospB genes are organized as a bicistronic operon and are subject to differential expression; Microbiology, 2002, 148, 2519-2529).

The new enzyme was named Shigella apyrase because it sequentially removes phosphates from nucleoside triphosphates, first yielding a diphosphate and therefrom the corresponding monophosphate (Bhargava T et al.; Babu MM et al.; Santepaola D et al.; loc. cit.).

Shigella apyrase is believed to be structurally similar to some known acidic phosphatases. However, it does not hydrolyze phosphate monoesters. Hence it was classified as a pyrophosphatase (Bhargava T et al.; Babu MM et al.; Santapaola D et al.; loc. cit.).

Shigella apyrase does not cleave dinucleotides containing an endogenic pyrophosphate such as NADP. Hence it was classified as an exo-pyrophospatase (Babu MM et al.; loc. cit.).

Shigella apyrase is a non-metal-dependant enzyme. It does not lose activity in the presence of complexing agents such as EDTA and does not gain significant activity in the presence of metal ions (Bhargava T et al.; Babu MM et al.; Santapaola D et al.; loc. cit.).

The lack of requirement for metal ions is unusual since generally pyrophosphatases are metal dependant enzymes (Babu MM et al.; loc. cit.).

Both Shigella spp. and EIEC produce Shigella apyrase. The Shigella apyrase gene (apy) is located on a plasmid associated with bacterial virulence. It is known that the apy gene is expressed during pathogenesis. There is evidence but no proof that Shigella apyrase is directly involved in pathogenesis of Shigella spp (Bhargava T et al.; Babu MM et al.; Santapaola D et al.; loc. cit.).

Shigellosis diseases caused by Shigella spp. and EIEC are among the most common pathogenic bacterial infections in humans.

The WHO reports burden of disease to include 165 million cases per year with an estimated 1.1 million deaths, mostly children younger than five years, elderly people and people with impaired immune defense.

The presence of microbial Shigella apyrase is limited to Shigella spp. and EIEC. Therefore, the presence of Shigella apyrase enzyme activity is indicative of the presence of said microbes (Bhargava T et al.; Babu MM et al.; Santapaola D et al.; loc. cit.).

The commonly used assay for Shigella apyrase is based on assaying inorganic phosphate that originates from Shigella apyrase catalyzed hydrolysis of pyrophosphate (Bhargava T et al.; Babu MM et al.; Santapaola D et al.; loc. cit.).

For the purpose of assaying Shigella apyrase, inorganic pyrophosphate or ATP is incubated with a sample in a biological buffer containing EDTA. Hydrolyzed pyrophosphate is measured by the addition of molybdate reagents such as those used for colorimetric detection of inorganic phosphates (Bhargava T et al.; Babu MM et al.; Santapaola D et al., loc. cit.).

EDTA serves to eliminate the activity of metal dependant phosphatases and pyrophosphatases and it can be used to induce cell lysis to release endo- or peri-plasmatic Shigella apyrase prior to assaying.

There are a number of established molybdate based phosphate assays. Often a molybdate such as ammonium molybdate is used in combination with a reducing agent such as ascorbic acid to yield molybdenum blue (Martin B et al. J. Biol. Chem., 1985, 260, 14932; Harder KW et al. Biochem. J. 1994, 298, 395; Campbell RB et al. J. Biol. Chem. 2003, 278, 36).

Other assays use molybdate and copper salts such as Malachite green oxalate (Martin B et al.; Harder KW et al.; Campbell RB et al.; loc. cit.).
However, most molybdate reagents give positive reaction with pyrophosphate. Because pyrophosphate is used as a substrate in the assay, the reactivity of molybdate reagents towards pyrophosphates is not desirable.

Recently, new molybdate reagents containing copper and iron sulfate instead of malachite green have been developed. It is claimed that these reagents do not react with pyrophosphate but still work for determination of inorganic phosphate (Babu MM et al.; loc. cit.).

However, other types of interference still exist. For example, it is well known that the phosphate determinations using molybdate reagents in clinical samples yield exaggerated values if samples are not freed of protein prior to assaying. It is believed that some proteins form precipitates with molybdates that inflate the consumption of reagent (Zaman Z, Sneyers L, Van Orshoven A, Blanckaert N and Mariën G: Elimination of Paraprotein Interference in Determination of Plasma Inorganic Phosphate by Ammonium Molybdate Method; Clinical Chemistry, 1995, 41, 609-614).

More importantly, the presently known assays for said pathogens do not provide for cheap, simple, robust and reliable detection. Shigellosis is most common in regions of the world that are short on laboratory infrastructure and lack staff skilled in laboratory techniques needed to perform the phosphate assay with molybdate reagents.

For instance, sample preparation requires the use of a centrifuge to remove solids and particles that cause turbid suspensions because turbidity of sample prevents meaningful colorimetric measurement or visual inspection.

### SUMMARY OF THE INVENTION

It is a primary objective of the present invention to provide improved assay methods for the detection and isolation of Shigella apyrase enzymes that do not have the above mentioned disadvantages.

The above and further objects are achieved by the detection method defined in claim 1, the assay method defined in claim 7, the detection kit defined in claim 8 and the use defined in claim 14. Preferred embodiments are defined in the dependent claims.

The methods of this invention are not based on assaying inorganic phosphates as described in the prior art, thus removing major limitations associated with such types of assays.

This invention provides a new simple, robust, cost effective and reliable assay method for Shigella apyrase producing pathogenic microbes such as *Shigella spp.* or EIEC bacteria which is badly needed to battle pandemic shigellosis.

Ideally, assay reagents are immobilized on a carrier, e.g. on a test strip. Such immobilization can be achieved by absorbing reagents on a carrier, for example by dissolving reagents in solvent and applying resulting solution to a solid followed by removal of solvent, or by chemical boding of reagents to solid phase polymeric materials.

Upon exposure to the crude sample, presence of Shigella apyrase and hence said bacterial pathogens are made detectable by color indication recognizable to the average human eye.

The inventive method is useful for the rapid detection of microbial pathogens producing Shigella apyrase. Expecting that cost can be kept at a substantially lower level as compared to the methods known in the art, the inventive method could allow mass screening for prevention and containment of Shigellosis.

In an equally desirable set-up, reagents are added to a microbial plating medium suitable for growth of *Shigella* and *E. Coli* bacteria colonies. After inoculation with sample and incubation, colonies producing Shigella apyrase become easily distinguishable from non-pathogenic microbes.

The assay is also useful to isolate pathogenic strains producing Shigella apyrase for genomic taxonomy or epidemiological investigation such as determining origin, development and spread of infectious outbreak.

Enzyme substrates for the detection of pyrophosphatases have been known for many years. Example therefor are the molecules with general structures **1** and **2**: 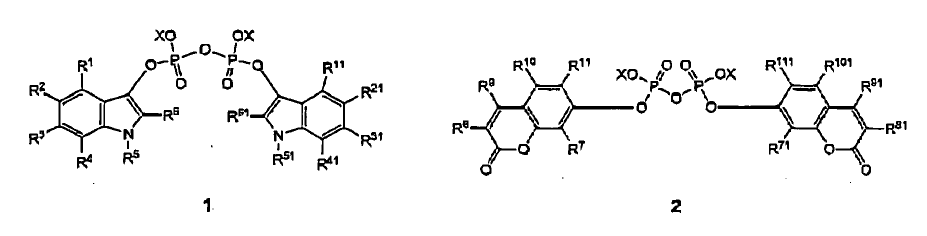 wherein each one of R¹, R², R³, R⁴, R¹¹, R²¹, R³¹ and R⁴¹ of said structure **1** is independently a hydrogen or a chromogenic substituent;
and wherein each one of R⁵, R⁶, R⁵¹ and R⁶¹ of said structure **1** is independently a hydrogen or a (C1-C4)alkyl or an arylmethyl;
and wherein each one of R⁷, R⁸, R⁹, R¹⁰, R⁷¹, R⁸¹, R⁹¹ and R¹⁰¹ of said structure 2 is independently a hydrogen or a chromogenic substituent;
and wherein each one of R¹¹ and R¹¹¹ of said structure **2** is independently a hydrogen or a (C1-C4)alkyl or an arylmethyl;
and wherein said chromogenic substituents include: halogen atoms, preferably fluorine, chlorine and bromine; C1-C4 alkyl groups, i.e. methyl, ethyl, propyl and butyl including the isomeric forms, optionally containing an oxygen atom in the alkyl chain; C1-C4 alkoxy; nitro; carboxy, C1-C4 carboxyalkyl; and cyano; optionally, the alkyl groups just mentioned may include one or more halogen atoms, preferably fluorine, chlorine and bromine.

While the organic endo-pyrophosphates **1** and **2** are shown as uncharged molecular structures wherein the pyrophosphate group includes two substituents X, it will be understood that the endo-pyrophosphates may be present as negatively charged ionic species with appropriate counter ions in solution. Accordingly, the above structures should be understood in the sense that X is hydrogen or a substituent or counter ion; preferably, X is hydrogen or a counter ion derived from organic or inorganic bases such as sodium hydroxide, potassium hydroxide, lithium hydroxide, ammonium hydroxide, diethylamine, triethylamine, tetramethylammonium hydroxide, tetraethylammonium hydroxide, cyclohexylamine, pyridine, piperidine, pyrrolidine, morpholine, N-methyl-morpholine, N-ethylmorpholine and p-toluidine.

Therefore, X is hydrogen or a substituent or counter ion, preferably sodium, potassium, lithium, ammonium, diethylamino, triethylamino, tetramethylammonium, tetraethylammonium, cyclohexylamino, pyridino, piperidino, pyrrolidino, morpholino, N-methyl-morpholino, N-ethyl-morpholino or p-toluidyl. It should be understood that other suitable counter ions may be taken into consideration.

It should also be understood that the pyrophosphate group may have two different substituents X' and X", wherein each one is according to the above definition for X. In particular, one of the substituents may be hydrogen and the other may be non-hydrogen.

Enzyme substrate structure **1** contains an indoxyl signalogen. The corresponding active signalogen which is released upon hydrolysis of the phosphate enzyme labile group by the detectable enzyme. The indoxyl active signalogen produces deeply colored indigo signalophore through oxidative dimerization.

Enzyme substrate structure **2** contains an umbelliferyl signalogen which produces fluorogenic active signalogen upon hydrolysis of the phosphate enzyme labile group. The active signalogen is deprotonated in alkaline solution to yield fluorescent signalophore.

There are two possible mechanisms that explain this observation (structure **1** used for elaboration):
(i) Hydrolysis of the pyrophosphate separates phosphate from an indoxyl signalogen, thus releasing the indoxyl active signalogen (Scheme 2-i).
(ii) Hydrolysis of the pyrophosphate splits the pyrophosphate in the middle into two mono phosphates. In this case, the indoxyl phosphate rather than the indoxyl is released as the active signalogen. In this case formation of signalophore would require a phosphatase activity that hydrolyses the indoxyl monophosphate subsequent to hydrolysis of the pyrophosphate (Scheme 2-ii).

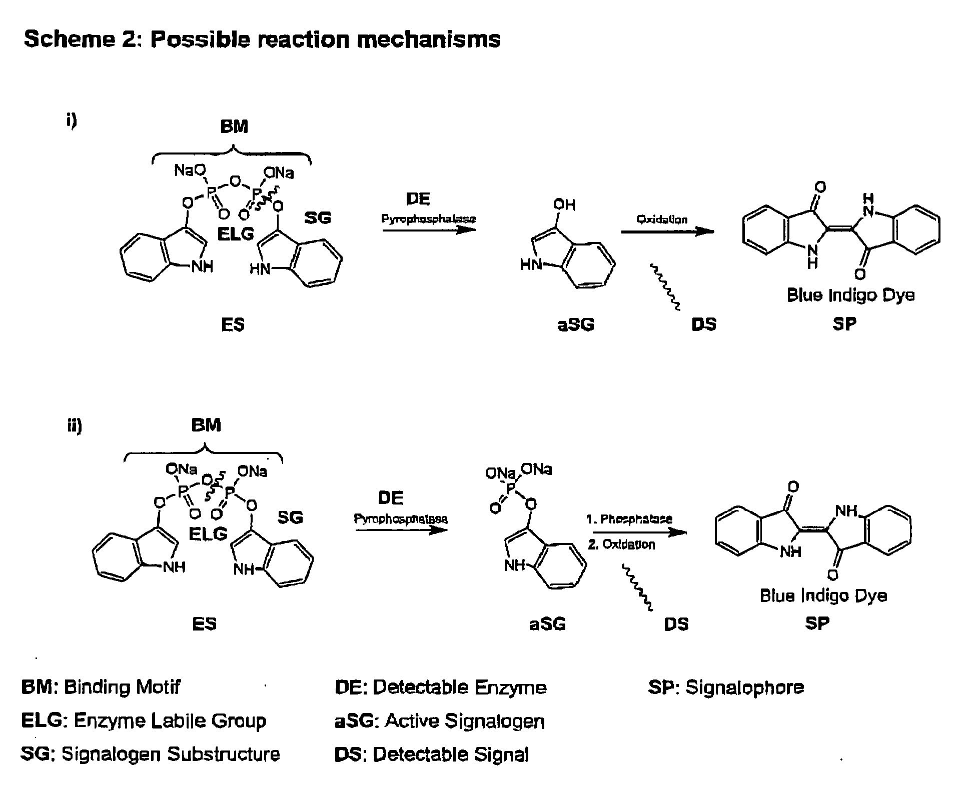

In practice, it is difficult to distinguish between the two reaction pathways because most pyrophosphatases display some phosphatase activity as well.

It is discussed in prior art that Shigella apyrase is different from other pyrophosphatases and does not promote the hydrolysis of monophosphate esters.

Therefore, we concluded that if enzyme substrates for pyrophosphatases are used to assay Shigella apyrase, an additional phosphatase activity must be present in order to generate signalophore and hence a detectable signal.

Such activity could be contributed by a phosphatase enzyme naturally present in the sample or by the addition of a specific reagent, preferably a phosphatase enzyme. It is essential that such agent or enzyme providing the conversion of the active signalogen in the signalophore is not by itself reactive towards the Shigella apyrase enzyme substrate, since otherwise this would lead to false positive results.

In prior art it was concluded that Shigella apyrase is an exo-pyrophosphatase. Consequently, endo-pyrophosphatase enzyme substrates such as **1a** to **1f, 2a** and **2b** shown hereinbelow were not expected to be susceptible to Shigella apyrase enzyme activity:

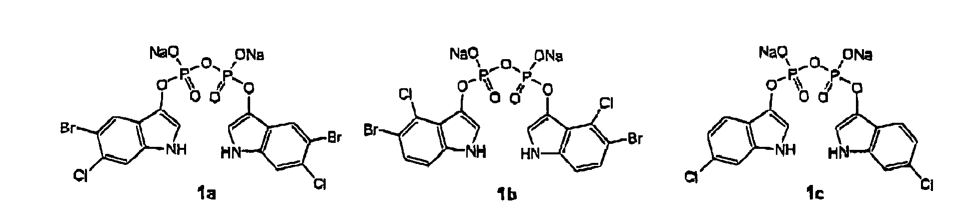

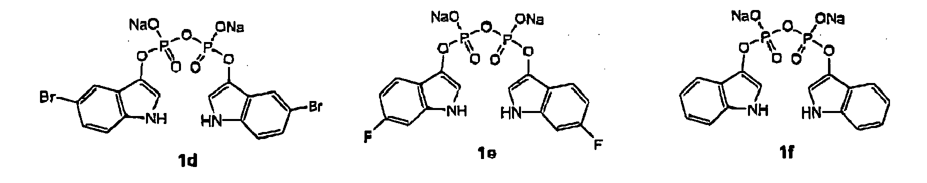

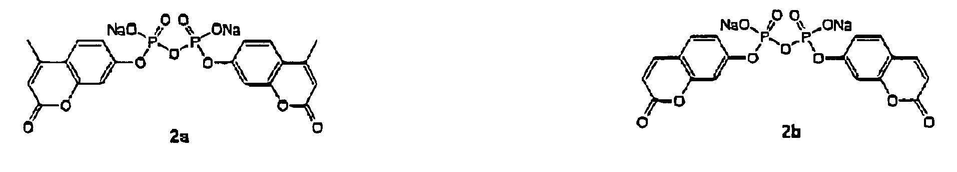

We discovered that this conclusion made in prior art is wrong. Consequently, Shigella apyrase should not be classified as an exo-pyrophosphatase as suggested in prior art. In contrary to accepted opinion, we found that Shigella apyrase displays significant endo-pyrophosphatase activity towards substrates such as **1a** to **1f, 2a** and **2b.**

Further, we found that of the two possible reaction pathways discussed above, the second one (ii) applies. In the absence of phosphatase activity, no signal can be detected because the active signalogen fails to convert into the respective signalophore.

Equally important is that we found that certain acidic phosphatases are not reactive towards endo-pyrophosphatase substrates such as **1** or **2**, which is a prerequisite to functioning of the assay.

Further, we confirmed that Shigella apyrase is not sensitive to chelating reagent such as EDTA and that other pyrophosphatases such as ATPase can be deactivated using such agents without compromising Shigella apyrase activity.

We also found that the acidic phosphatase used as an auxiliary reagent to convert the active signalogen into the signalophore is not affected by chelators such as EDTA.

EDTA is useful to eliminate non Shigella apyrase related enzyme activity that may interfere with the assay. In addition, it can be used to induce cell lysis to release peri- or endoplasmatic Shigella apyrase prior to assay.

In summary, we found that endo-pyrophosphatase enzyme substrates such as **1** and **2** are well suited to assay Shigella apyrase in combination with a reagent phosphatase enzyme. A chelator such as EDTA or the addition of Tris-Buffer may be beneficial to eliminate undesired enzyme activity and to induce cell lysis to make available Shigella apyrase enzyme.

Based on our observations, we expect that most pyrophosphates that contain signalogens which upon activation by Shigella apyrase produce active signalogens that are substrates to mono-phosphatases can be used in the present Shigella apyrase assay.

In a first set of preferred embodiments, the objective is rapid detection of Shigella apyrase producing microorganisms.

In one of these preferred embodiments, bis-(5-bromo-6-chloroindolyl-3)-pyrophosphate **1a** is used as chromogenic Shigella apyrase enzyme substrate in the assay.

In a more preferred embodiment, said substrate is loaded onto a solid material such as cellulose, silica gel or aluminium oxide possibly shaped into plates, strips or discs that may be supported by a suitable carrier material such as glass, plastic or aluminium.

In a even more preferred embodiment, acidic phosphatase is loaded onto the solid material in addition to said enzyme substrate.

It may be beneficial to use additional reagents that promote formation of signalophore from indoxyl active signalogens such as tetrazolium salts or phenylenediamine compounds that preferably are immobilized on said solid in addition.

In a most preferred embodiment, a zone of solid material loaded with the reagents, henceforth called "reagent region" is adjacent to a zone of solid material that serves to apply the sample, henceforth called "sampling zone". Application of the sample may occur by dipping the sampling zone into the liquid sample or spotting sample on the sampling zone. The design of the device ensures that after application of sample the liquid diffuses from the sampling zone into the adjacent reagent zone aided by capillary effect. The process of diffusing serves to eliminate solid particles and to avoid extraction from reagent immobilized on solid material into the sample.

In a further embodiment, bis-(3-indoxy)-pyrophosphates are used as electrogenic substrates.

In such set-up, formation of indigo is followed by electrochemical reduction to leuco-indigo rather than optical spectroscopy of visual inspection. The flow of current correlates with the amount of indigo formed as it is described in the literature (Yeo WS et al., loc. cit.).

In an additional embodiment, fluorogenic pyrophosphatase substrates such as **2** are used in the assay.

In an other embodiment, luminogenic substrates such as dioxethanes are used in the assay (Giri BP, loc. cit.).

In a alternative second set of preferred embodiments, the objective is isolation of Shigella apyrase producing species.

In a preferred embodiment, bis-(5-bromo-6-chloroindolyl-3)-pyrophosphate **1a** or other precipitating enzyme substrate for Shigella apyrase together with acidic phosphatase is added to standard Shigella plating media. During incubation, some colonies turned purple in color. The color was found to be highly indicative of Shigella apyrase producing species. Selective picking of colored coulter allowed isolation of pathogenic organisms for the purpose of taxonomy and epidemiological study.

The same effect can be achieved by the addition of enzyme substrate to plating media and staining grown colonies with acidic phosphatase after incubation.

### EXAMPLES

### Example 1: Preparation of substrates

Synthesis of enzyme substrates **1e, 1f, 2a** and **2b** was performed according or in analogy to published procedures (Tsou KC, Su HCF, Rabiger DJ, Heymann HA and Seligman AM: Synthesis of 3-Indolyl and 5-Bromo-3-indolyl Phosphate for Histochemical Demonstration of Alkaline Phosphatase. J. Med. Chem., 1967, 10, 662-664).

### Example 2: Assay

Stock solution: 20 mM EDTA in pH 7.5 buffer containing 0.2 g/l enzyme substrate **1a**.

A sample is inoculated in 3 ml of appropriate nutrient broth and incubated at 37°C for 16h. Then the sample is pelleted by means of a centrifuge and washed with 0.9% saline containing 1 mM CaCl₂.

The pellet is suspended 0.2 ml stock solution and incubated at 37°C for another 30 min. The sample is centrifuged again but supernatant is retained this time. Sample pH value is adjusted to below 6.5 before a drop of commercial acidic phosphatase is added. Appearance of purple color after 30 min at 37°C indicates positive result.

### Example 3: Assay

Stock solution: 40 mM EDTA in pH 7.5 buffer containing 0.2 g/l enzyme substrate **1a.**

A sample is inoculated in 3 ml of appropriate nutrient broth and incubated at 37° for 16h. An 0.1 ml aliquot is taken from the sample and diluted with 0.1 ml stock solution. The sample is incubated for another 30 min at 37°C before the pH is adjusted to below 6.5 and a drop of commercial acidic phosphatase is added. A magenta color appear after 30 min at 37°C. If the sample is very dark and turbid it is spotted on cellulose filter paper. The appearance of a purple halo around the spot indicates positive result.

### Example 4: Immobilization of reagents

Strips of cellulose filter paper about 0.5 x 3 cm in size are dipped vertically into saturated solution of enzyme substrate **1a** in 80% aq. methanol such that the liquid rises within the paper to a level of about 1 cm below the upper edge of the strip. The strips are allowed to dry in the open air before about 1 microlitre acidic phosphatase is applied to the reagent zone of every strip that contains the enzyme substrate. A hydrophobic zone separates the reagent zone from the zone for handling so as to avoid contamination of the latter. Immobilization of enzyme substrate can be improved by using a diluted wallpaper-paste as solvent and further loading as described.

### Example 5: Assay

Stock solution: 40 mM EDTA in pH 7.5 buffer.

A sample is inoculated in 3 ml of an appropriate nutrient broth and incubated at 37° for 16h. An 0.1 ml aliquot is taken from the sample and diluted with 0.1 ml stock solution. The sample is incubated for another 30 min at 37°C before the pH is adjusted to below 6.5. A drop of the sample is spotted on the reagent zone of the test strips prepared as described in example 4. Appearance of purple color indicates positive result.

### Example 6: Immobilization of Reagents

Same procedure as in example 4, but using acidic aluminium oxide supported by inert backing material instead of paper strips.

### Example 7: Assay

Stock solution: 40 mM EDTA in pH 7.5 buffer.

A sample is inoculated in 3 ml of nutrient broth and incubated at 37° for 16h. An 0.1 ml aliquot is taken from the sample and diluted with 0.1 ml stock solution. The sample is incubated for another 30 min at 37°C. A drop of the sample is spotted on the sample zone of the test strips prepared as described in example 6. Appearance of purple color indicates positive result.

### Example 8: Assay

Nutrient agar (pH7.5) is supplemented with 0.2 g enzyme substrate/l, 2 mM EDTA and 0.02 g acidic phosphatase/l. Magenta colored Shigella spp. colonies appear after 24 h of incubation at 37°C.

### Example 9: Assay

Nutrient agar (pH7.5) is supplemented with 0.2 g enzyme substrate and 2 mM EDTA. The grown Shigella spp. colonies appear magenta-colored after staining with acidic phosphatase (2 mg/10 ml dist. water).

## Claims

1. A method of detecting Shigella apyrase enzyme comprising the steps of:
a) contacting a sample suspected of containing said Shigella apyrase enzyme with an endo-pyrophosphatase substrate, said substrate comprising an endo pyrophosphate group that is susceptible to cleavage by said enzyme, said cleavage yielding a signalogen species comprising a monophosphate group, said signalogen species being susceptible to phosphatase-induced formation of a signalophore species;
b) providing a phosphatase activity so as to convert said signalogen species into a signalophore species;
c) monitoring for a signal from said signalophore species as an indication of the presence of said Shigella apyrase enzyme.

2. The method of claim 1, wherein said substrate comprises at least one 3-indoxyl moiety of formula (3) 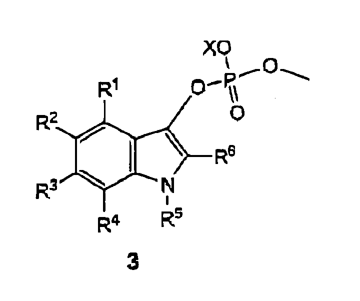 wherein each one of R¹, R², R³ and R⁴ is independently a hydrogen or a chromogenic substituent;
wherein said chromogenic substituents include: halogen atoms, particularly fluorine, chlorine and bromine; C1-C4 alkyl groups, particularly methyl, ethyl, propyl and butyl including the isomeric forms, optionally containing an oxygen atom in the alkyl chain; C1-C4 alkoxy; nitro; carboxy. C1-C4 carboxyalkyl; and cyano; said alkyl groups optionally including one or more halogen atoms, particularly fluorine, chlorine or bromine;
and wherein each one of R⁵ and R⁶ is independently a hydrogen or a (C1-C4)alkyl or an arylmethyl;
and wherein X is hydrogen or a substituent or counter ion, particularly sodium, potassium, lithium, ammonium, diethylamino, triethylamino, tetramethylammonium, tetraethylammonium, cyclohexylamino, pyridino, piperidino, pyrrolidino, morpholino, N-mathyl-morpholino, N-othyl-morpholino or p-toluidyl.

3. The method of claim 1, wherein said substrate comprises at least one umbelliferyl moiety of formula (4) 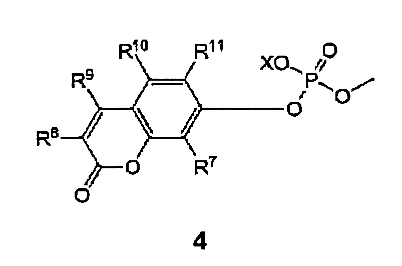 wherein each one of R⁷, R⁸, R⁹ and R¹⁰ is independently a hydrogen or a chromogenic substituent;
wherein said chromogenic substituents include: halogen atoms, particularly fluorine, chlorine and bromine; C1-C4 alkyl groups, particularly methyl, ethyl, propyl and butyl including the isomeric forms, optionally containing an oxygen atom in the alkyl chain: C1-C4 alkoxy: nitro; carboxy, C1-C4 carboxyalkyl; and cyano; said alkyl groups optionally including one or more halogen atoms, particularly fluorine, chlorine or bromine;
and wherein R¹¹ is hydrogen or a (C1-C4)alkyl or an arylmethyl;
and wherein X is hydrogen or a substituent or counter ion, particularly sodium, potassium, lithium, ammonium, diethylamino, triethylamino, tetramethylammonium, tetraethylammonium, cyclohexylamino, pyridino, piperidino, pyrrolidino, morpholino, N-mathyl-morpholino, N-ethyl-morpholino or p-toluidyl.

4. The method of claim 1, wherein said substrate is selected from the group consisting of the compounds with formulae (1a), (1b), (1c), (1d), (1e), 1(f), (2a) and (2b):

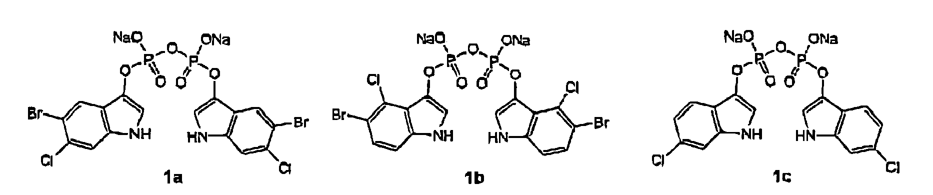

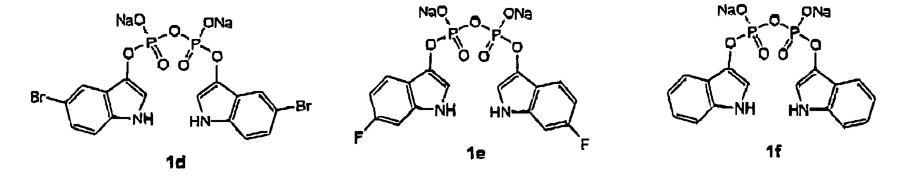

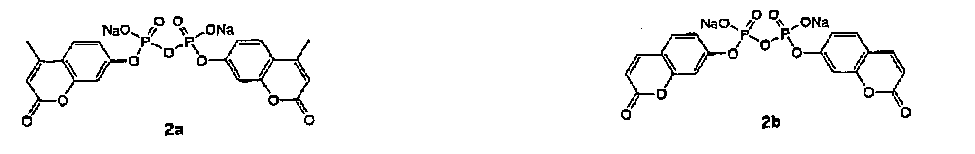

5. The method of claim 1, wherein step a) further comprises adding a metal chelator, particularly EDTA.

6. The method of claim 1, wherein said signal monitoring comprises detecting a change in optical properties.

7. An assay method for detection of the presence of *Shigella spp,* or enteroinvasive *E. coli,* including the step of detecting Shigella apyrase enzyme according to the method of any one of claims 1 to 6.

8. A kit for detecting Shigella apyrase enzyme, comprising:
a) an endo-pyrophosphatase substrate, said substrate comprising an endo pyrophosphate group that is susceptible to cleavage by said enzyme, said cleavage yielding a signalogen species comprising a monophosphate group, said signalogen species being susceptible to phosphatase-induced formation of a signalophore species;
b) means for monitoring for a signal from said signalophore species as an indication of the presence of said Shigella apyrase enzyme.

9. The kit of claim 8, further comprising a phosphatase enzyme, particularly acidic phosphatase.

10. The kit of claim 8 or 9, wherein said substrate comprises at least one 3-indoxyl moiety of formula (3) 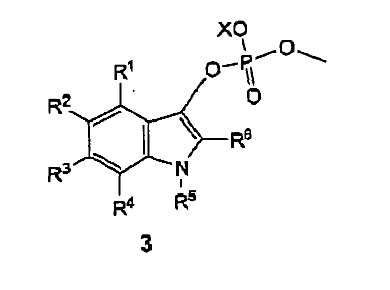 wherein each one of R¹, R², R³ and R⁴ is independently a hydrogen or a chromogenic substituent;
wherein said chromogenic substituents include: halogen atoms, particularly fluorine, chlorine and bromine; C1-C4 alkyl groups, particularly methyl, ethyl, propyl and butyl including the isomeric forms, optionally containing an oxygen atom in the alkyl chain; C1-C4 alkoxy; nitro; carboxy, C1-C4 carboxyalkyl; and cyano; said alkyl groups optionally including one or more halogen atoms, particularly fluorine, chlorine or bromine;
and wherein each one of R⁵ and R⁶ is independently a hydrogen or a (C1-C4)alkyl or an arylmethyl;
and wherein X is hydrogen or a substituent or counter ion, particularly sodium, potassium, lithium, ammonium, diethylamino, triethylamino, tetramethylammonium, tetraethylammonium, cyclohexylamino, pyridino, piperidino, pyrrolidino, morpholino, N-methyl-morpholino, N-athyl-morpholino or p-toluidyl.

11. The kit of claim 8 or 9, wherein said substrate comprises at least one umbelliferyl moiety of formula (4) 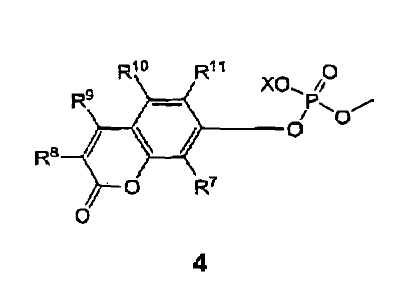 wherein each one of R⁷, R⁸, R⁹ and R¹⁰ is independently a hydrogen or a chromogenic substituent;
wherein said chromogenic substituents include: halogen atoms, particularly fluorine, chlorine and bromine; C1-C4 alkyl groups, particularly methyl, ethyl, propyl and butyl including the isomeric forms, optionally containing an oxygen atom in the alkyl chain; C1-C4 alkoxy; nitro; carboxy, C1-C4 carboxyalkyl; and cyano; said alkyl groups optionally including one or more halogen atoms, particularly fluorine, chlorine or bromine;
and wherein R¹¹ is hydrogen or a (C1-C4)alkyl or an arylmethyl;
and wherein X is hydrogen or a substituent or counter ion, particularly sodium, potassium, lithium, ammonium, diethylamino, triethylamino, tetramethylammonium, tetraethylammonium, cyclohexylamino, pyridino, piperidino, pyrrolidino, morpholino, N-methyl-morpholino, N-ethyl-morpholino or p-toluidyl.

12. The kit of claim 8 or 9, wherein said substrate is selected from the group consisting of the compounds with formulae (1a), (1b), (1c), (1d), (1e), 1(f), (2a) and (2b):

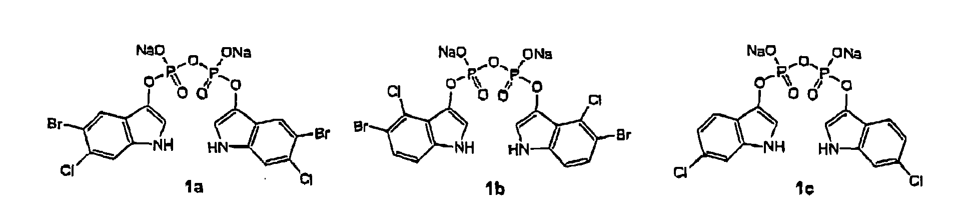

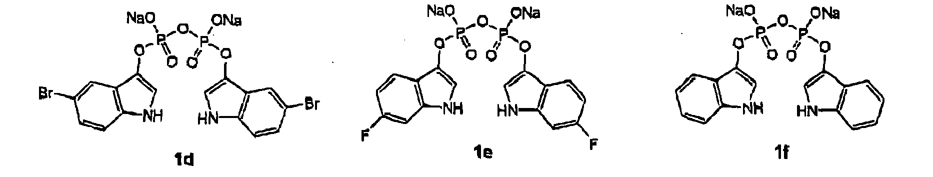

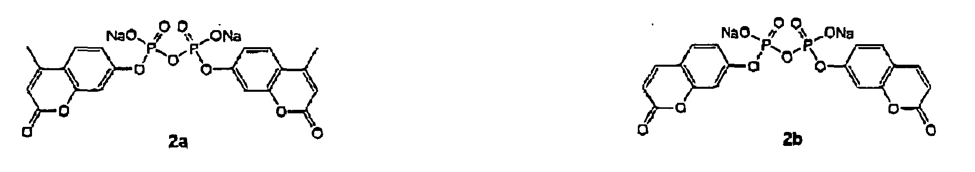

13. The kit of any one of claims 8 to 12, wherein reagents are immobilized on solid phase.

14. Use of an endo-pyrophosphatase enzyme substrate for detecting Shigella apyrase enzyme.
